# EUROPÄISCHE PATENTSCHRIFT

(11) **EP 1 321 119 B1**
(45) Veröffentlichungstag und Bekanntmachung des Hinweises auf die Patenterteilung: **20.12.2006**
(21) Anmeldenummer: 02023309.4
(22) Anmeldetag: 17.10.2002
(51) Int. Cl.: A61F 15/00, A47K 10/24, B65H 1/00, B65D 83/08

(54) **Windelspender**
Diaper dispenser
Distributeur de couches

(30) Priorität: 14.12.2001 DE 10161617
(43) Veröffentlichungstag der Anmeldung: 25.06.2003
(73) Patentinhaber: Kohne, Michael, 79286 Glottertal (DE)
(72) Erfinder: Kohne, Michael, 79286 Glottertal (DE); Finken, Martina, 70000 Auxon les Vesoul (FR)
(74) Vertreter: Prietsch, Reiner

(56) Entgegenhaltungen:
- DE-U- 20 103 539
- DE-U- 29 607 763
- US-A- 3 156 378
- US-A- 4 573 608

## Beschreibung

Die Erfindung betrifft einen Windelspender, bestehend aus einem zur Aufnahme liegend gestapelter Windeln bestimmten Behälter mit einer Wand, einem Boden und einer dazwischen befindlichen Entnahmeöffnung, über welche die zuunterst liegende Windel herausziehbar ist.

Konfektionierte, zum einmaligen Gebrauch bestimmte sogenannte Wegwerfwindeln gelangen etwa rechteckig gefaltet und einzeln in Stapeln zu beispielsweise 50 Stück verpackt in den Verkehr. Baumwollwindeltücher werden üblicherweise ebenfalls auf ein etwa rechteckiges Format zusammengefaltet und gestapelt zum Gebrauch bereitgehalten. Die in der Regel auf dem Wickeltisch abgesetzten Windelstapel können insbesondere bei der Entnahme einer Windel leicht umstürzen.

Dies vermeidet ein zur Aufnahme von Wegwerfwindeln bestimmter Windelspender der einleitend angegebenen Gattung, der aus der DE 296 07 763 U bekannt ist. Die Entnahmeöffnung ist als Querschlitz am unteren Ende einer der beiden Seitenwände des Behälters ausgebildet und wird unten von dem entsprechenden, eine kleine Ausnehmung aufweisenden Rand des Behälterbodens sowie oben von dem Unterrand der entsprechend gekürzten Seitenwand begrenzt. Die Seitenwand hat . außerdem einen in den Querschlitz mündenden Längsschlitz zur Füllstandskontrolle. Diese Konstruktion zur Entnahme der jeweils zuunterst liegenden Windel ist wenig praktikabel.

Ein ganz ähnlich konstruierter Windelspender mit den gleichen Nachteilen, der zudem von oben mit den Windeln beschickt werden muss, ist aus der DE 296 21 781 U bekannt.

Ein weiterer Windelspender ist aus der DE 201 03 539 U1 bekannt. Auch in diesen Windelspender müssen die Windeln von oben eingefüllt werden, was bei einem z.B. 50 Windeln umfassenden Windelstapel mit dem Risiko verbunden ist, dass der Stapel auseinanderfällt, so dass eine oder mehrere Windeln nicht mehr plan liegen sondern sich verkanten oder knicken. Dadurch wird sowohl die Entnahme der Windeln als auch das geordnete Nachrutschen des Stapels nach jeder Entnahme erschwert oder sogar blockiert. Die Entnahme erfolgt über eine in der Behälterwand angeordnete und somit horizontal gerichtete Öffnung, unterstützt von einem schrägen Zwischenboden als zusätzlichem Teil, das einer gesonderten Befestigung bedarf.

Der Erfindung liegt die Aufgabe zugrunde, einen die Lagerung und die Entnahme der Windeln vereinfachenden Windelspender zu schaffen.

Diese Aufgabe ist erfindungsgemäß durch einen Windelspender mit den im Patentanspruch 1 angegebenen Merkmalen gelöst.

Der den Windelspender bildende Behälter hat einen an das sehr ähnliche Format von gefalteten Wegwerfwindeln auch unterschiedlicher Hersteller angepaßten Innenquerschnitt. Auf das gleiche Format werden gewöhlich auch konventionelle Baumwollwindeltücher gefaltet. Die Höhe des Behälters kann so bemessen sein, daß er zwei handelsübliche Verpackungseinheiten zu z.B. 50 Stück Wegwerfwindeln übereinander gestapelt aufnehmen kann. Der Behälter kann Füße oder ein Fußgestell haben, so daß seine Entnahmeöffnung von unten zugänglich ist. Der Behälter ist jedoch in erster Linie für die Befestigung an einer Wand konstruiert. Dies hat den Vorteil, daß der Platzbedarf für das Bereithalten der Windeln auf dem Wickeltisch wegfällt oder sich zumindest verringert. Der wichtigste Vorteil des Windelspenders besteht indessen darin, daß sich die jeweils zuunterst befindliche Windel gezielt mit einem einzigen Handgriff nach vorne und unten aus dem Windelspender herausziehen läßt. Die Entnahmeöffnung umfaßt einerseits eine Eingrifföffnung, andererseits eine sich zumindest nahezu über die gesamte Behälterbreite erstreckende Ausnehmung im Boden des Behälters. Die Eingrifföffnung ist am Unterrand der Vorderwand des Behälters vorzugsweise mittig angeordnet und am besten für den Eingriff mit dem Daumen ausgelegt, so daß die Finger unter die zuunterst liegende Windel im Bereich der Bodenausnehmung zu liegen kommen. Dabei sorgt die Einziehung im Bereich der Eingrifföffnung dafür, dass die unteren Windeln in Richtung auf die Rückwand des Behälters leicht gestaucht werden und die unterste Windel besser zwischen Daumen und Fingern ergriffen werden kann.

Die Eingrifföffnung hat vorzugsweise eine die Dicke einer gestapelten Windel übersteigende Höhe (Anspruch 2). Jedoch ist ihre Höhe zweckmäßig deutlich geringer als die doppelte Dicke einer gestapelten Windel (Anspruch 3), damit der eingreifende Daumen gewissermaßen selbsttätig die zuunterst liegende von der zweituntersten Windel trennt und erstere dann durch leichten Druck nach unten über die Ausnehmung im Boden des Behälters herausgezogen werden kann.

Daß der Windelvorrat zu Ende geht, ist dann leicht erkennbar, wenn die Vorderwand Sichtfenster hat (Anspruch 4). Ein Sichtfenster kann genügen. Stattdessen können mehrere Sichtfenster in unterschiedlicher Höhe oder ein langgestrecktes Sichtfenster angeordnet werden.

Zweckmäßig hat der Behälter näherungsweise die Form eines geraden, vierflächigen Prismas (Anspruch 5), das aus gestalterischen Gründen etwas abgerundete Formen haben kann.

Bevorzugt hat der Behälter zur Anpassung seines inneren Querschnitts an Windelstapel unterschiedlicher Formate eine Innenwand, die parallel zu der Rückwand des Behälters in Richtung auf die Vorderwand verstellbar und feststellbar ist (Anspruch 6). Die Feststellung der Innenwand kann insbesondere durch elastische Ausbildung und das Zusammenwirken von zur Rückwand des Behälters parallelen Rippen im Behälterboden und in der Behälterdeckfläche oder in den Behälterseitenwänden sowie entsprechende Rastnasen an den entsprechenden Rändern der Innenwand erreicht werden.

Um sicherzustellen, daß die gefalteten Windeln in dem sich mit jeder Windelentnahme nach unten bewegenden und seine Höhe verringernden Stapel plan liegen bleiben, kann eine Beschwerung des Windelstapels durch einen plattenförmigen Körper oder dergleichen zweckmäßig sein. (Anspruch 7).

Vorteilhaft kann mindestens eine der Behälterseitenwände zur Befestigung einer Ablagekonsole ausgebildet sein (Anspruch 8), auf der z.B. Puder- oder Cremebehälter abgestellt werden können.

In der Zeichnung ist der Windelspender nach der Erfindung in einer beispielsweise gewählten Ausführungsform, zum Teil vereinfacht, dargestellt. Es zeigt:
- Fig. 1: eine perspektivische Ansicht,
- Fig. 2: eine Explosionsdarstellung,
- Fig. 3 und 4: schematische Seitenansichten des gefüllten Behälters, zur Veranschaulichung der Entnahme.

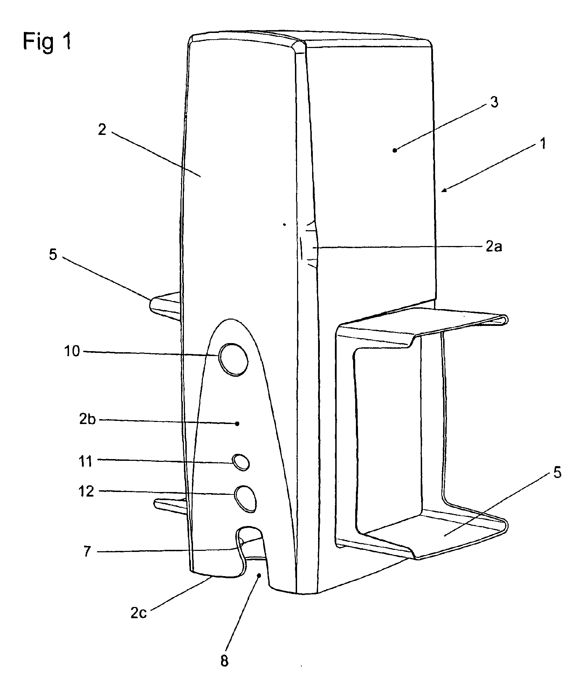

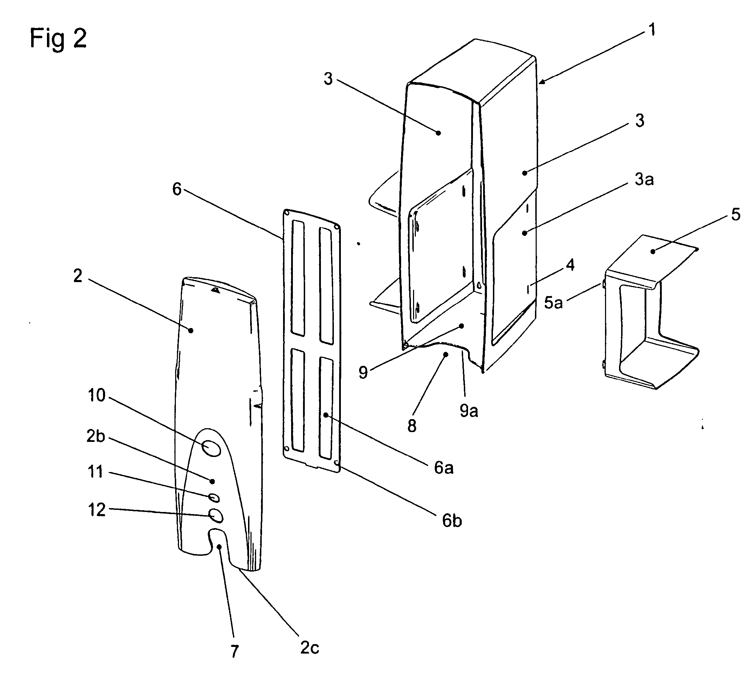

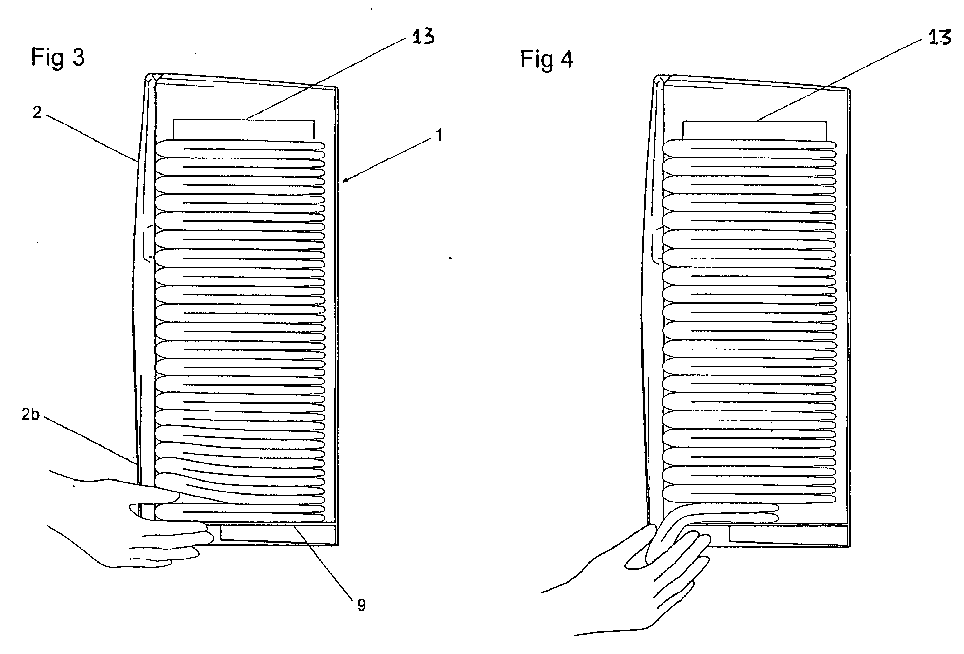

Der in den Figuren 1 und 2 dargestellte Windelspender umfaßt einen näherungsweise parallelepipedischen Behälter 1, vorzugsweise in Form eines Kunststofftiefziehteiles, mit Innenabmessungen zur Aufnahme beispielsweise eines Stapels von einmal gefalteten Wegwerfwindeln (vgl. z.B. Figur 3). Der Behälter hat eine Vorderwand, die als Deckel 2 ausgebildet ist, der über Rastnasen wie 2a in Fig. 1 auf den Behälter 1 aufschnappbar ist. Die Behälterseitenwände 3 haben vertiefte Planflächen 3a mit Schlitzen 4 zum Einhängen von mit korrespondierenden Haken 5a ausgebildeten, seitlichen Konsolen 5.

In dem Behälter 1 befindet sich eine Innenwand 6, die parallel zu der Rückwand des Behälters in Richtung auf den Deckel 2 verstellbar und feststellbar ist, um den inneren Querschnitt des Behälters 1 an Windelstapel anpassen zu können, deren Format kleiner als das durch die Tiefe des Behälters vorgegebene, maximale Format ist. Die Innenwand 6 hat lediglich zur Gewichts- und Materialersparnis vier Längsschlitze 6a sowie vier Löcher 6b, über welche nicht dargestellte deckungsgleiche Löcher in der Behälterrückwand zugänglich sind, die zur Aufnahme von Schrauben bestimmt sind, mittels welcher der Behälter 1 an einer Wand befestigbar ist. Zur Festlegung der Innenwand 6 kann der Behälter an geeigneten Stellen Rippen haben, in welche die Ränder der Innenwand 6 elastisch einrasten.

Der Deckel 2 hat eine von seinem Unterrand ausgehende, muldenartige Einziehung 2b, die bei der halben Höhe des Deckels 2 ausläuft. In diesem Bereich sind von oben nach unten eine Vertiefung 10, die zur Anbringung eines Markenzeichens dienen kann, und zwei Fenster 11 und 12 angeordnet. Diese Fenster können einfache Löcher oder transparente Kunststoffscheiben sein. Sie ermöglichen eine Füllstandskontrolle und sind somit überflüssig, wenn der Behälter 1 oder zumindest der Deckel 2 aus einem zumindest durchscheinenden Kunststoff sind.

Für die Funktionalität besonders wichtig ist die Gestaltung der Entnahmeöffnung für die Windeln. Die Entnahmeöffnung umfaßt zwei zusammenwirkende Ausnehmungen, nämlich einerseits eine Eingrifföffnung 7, die sich in Form eines mittigen Schlitzes vom Unterrand 2c des Deckels 2 bis auf eine Höhe erstreckt, die etwas größer als die Dicke einer durchschnittlichen, gefalteten Windel entspricht. Die Eingrifföffnung 7, die sich im tiefsten Bereich der muldenförmigen Einsenkung 2b befindet, ist ergonomisch so geformt, daß sich für den Benutzer die Einführung des Daumens seiner rechten oder linken Hand anbietet. Dann kommen die Finger gemäß Figur 3 unter der zuunterst liegenden Windel in eine den zweiten Teil der Entnahmeöffnung bildende Ausnehmung 8 zu liegen, die im Boden 9 des Behälters 1 als Rücksprung seines vorderwandseitigen Randes 9a ausgebildet ist. Der Rücksprung erstreckt sich von Seitenwand 3 zu Seitenwand 3, zweckmäßig, aber nicht notwendigerweise, so, daß er in der Mitte, in Höhe der Eingrifföffnung 7, seine größte Tiefe bzw. Öffnungsweite hat. Seine minimale Tiefe und damit die minimale Breite der Ausnehmung 8 im Boden 9 des Behälters 1 muß aber etwa gleich der Stärke der dicksten handelsüblichen bzw. aus dem Behälter zu entnehmenden Windel sein.

Die Figuren 3 und 4 veranschaulichen den Entnahmevorgang. Der über die Eingrifföffnung eingeführte Daumen trennt die zuunterst liegende Windel von der zweituntersten Windel. Die zuunterst liegende Windel wird zwischen Daumen und den Fingern ergriffen, in dem ergriffenen Bereich etwas nach unten abgekippt und dann schräg nach vorne/unten über die schlitzförmige Ausnehmung in dem Boden 9 des Behälters 1 herausgezogen. Eine Beschwerungsplatte 13 sorgt dafür, daß der Windelstapel zuverlässig nachrutscht, und die Windeln dabei ihre plane Lage beibehalten.

## Patentansprüche

1. Windelspender, bestehend aus einem zur Aufnahme liegend gestapelter Windeln bestimmten Behälter (1) mit einer Wand, einem Boden (9) und einer dazwischen befindlichen Entnahmeöffnung, über welche die zuunterst liegende Windel herausziehbar ist, wobei die Entnahmeöffnung sich in der Vorderwand (2) des Behälters (1) befindet und eine sich vom Unterrand (2c) der Vorderwand (2) nach oben erstreckende, etwa schlitzförmige Eingrifföffnung (7) und eine Ausnehmung (8) umfasst, die als Rücksprung des vorderwandseitigen Randes (9a) des Bodens (9) des Behälters (1) ausgebildet ist, **dadurch gekennzeichnet dass** die Vorderwand (2) im Bereich der Eingrifföffnung (7) eine Einziehung (2b) hat, und dass die Vorderwand (2) als aufschnappbarer Deckel des Behälters (1) ausgebildet ist.

2. Windelspender nach Anspruch 1, **dadurch gekennzeichnet, daß** die Eingrifföffnung (7) eine die Dicke einer gestapelten Windel übersteigende Höhe hat.

3. Windelspender nach Anspruch 1 oder 2, **dadurch gekennzeichnet, daß** die Eingrifföffnung (7) eine Höhe kleiner als die doppelte Dicke einer gestapelten Windel hat.

4. Windelspender nach einem der Ansprüche 1 bis 3, **dadurch gekennzeichnet, daß** die Vorderwand (2) Sichtfenster (11,12) hat.

5. Windelspender nach einem der Ansprüche 1 bis 4, **dadurch gekennzeichnet, daß** der Behälter (1) näherungsweise die Form eines geraden, vierflächigen Prismas hat.

6. Windelspender nach einem der Ansprüche 1 bis 5, **dadurch gekennzeichnet, daß** der Behälter (1) zur Anpassung seines inneren Querschnitts an Windelstapel unterschiedlicher Formate eine Innenwand (6) hat, die parallel zu der Rückwand des Behälters (1) in Richtung auf die Vorderwand (2) verstellbar und feststellbar ist.

7. Windelspender nach einem der Ansprüche 1 bis 6, **gekennzeichnet durch** einen plattenförmigen Körper (11) zur Beschwerung des Windelstapels.

8. Windelspender nach einem der Ansprüche 1 bis 7, **dadurch gekennzeichnet, daß** mindestens eine der Behälterseitenwände zur Befestigung einer Ablagekonsole (5) ausgebildet ist.

## Claims

1. A diaper dispenser, consisting of a container (1) determined for receiving stacked lying diapers and comprising a wall, a floor (9) and an interposed removal opening, through which the lowermost diaper can be pulled out, with the removal opening being located in the front wall (2) of the container (1) and comprising an approximately slit-shaped engagement opening (7) and a recess (8), which engagement opening extending upwardly from the bottom edge (2c) of the front wall (2), and which recess is arranged as a setback of the edge (9a) on the side of the front wall of the floor (9) of the container (1), **characterized in that** the front wall (2) comprises a retraction (2b) in the region of the engagement opening (7) and that the front wall (2) is arranged as a lid of the container (1) which can be flipped open.

2. A diaper dispenser according to claim 1, **characterized in that** the engagement opening (7) has a height which exceeds the thickness of a stacked diaper.

3. A diaper dispenser according to claim 1 or 2, **characterized in that** the engagement opening (7) has a height which is smaller than twice the thickness of a stacked diaper.

4. A diaper dispenser according to one of the claims 1 to 3, **characterized in that** the front wall (2) has an inspection window (11, 12).

5. A diaper dispenser according to one of the claims 1 to 4, **characterized in that** the container (1) has the approximate shape of a straight, tetrahedral prism.

6. A diaper dispenser according to one of the claims 1 to 5, **characterized in that** the container (1), for the adjustment of its inner cross section to stacks of diapers of different formats, has an inner wall (6) which parallel to the rear wall of the container (1) is adjustable in the direction towards the front wall (2) and fixable.

7. A diaper dispenser according to one of the claims 1 to 6, **characterized by** a plate-like body (11) for putting weight on the stack of diapers.

8. A diaper dispenser according to one of the claims 1 to 7, **characterized in that** at least one of the container side walls is arranged for fastening a deposit console (5).

## Revendications

1. Distributeur de couches, constitué d'un récipient (1) destiné à recevoir des couches empilées horizontalement et comportant une paroi, un fond (9) et une ouverture de prélèvement interposée par laquelle la couche inférieure peut être prélevée, l'ouverture de prélèvement se situant dans la paroi avant (2) du récipient (1) et comportant un orifice d'introduction (7) d'un doigt en forme approximative de fente s'étendant vers le haut depuis le bord inférieur (2c) de la paroi avant (2) et un évidement (8) conformé en forme de décrochement du bord (9a) côté paroi avant du fond (9) du récipient (1), **caractérisé en ce que** la paroi avant (2) possède, à la hauteur de l'orifice d'introduction (7), une partie rentrante (2b) et que la paroi avant (2) est conformée en forme de couvercle mû par un ressort du récipient (1).

2. Distributeur de couches selon la revendication 1, **caractérisé en ce que** l'orifice d'introduction (7) a une hauteur supérieure à l'épaisseur d'une couche empilée.

3. Distributeur de couches selon la revendication 1 ou 2, **caractérisé en ce que** l'orifice d'introduction (7) a une hauteur inférieure à la double épaisseur d'une couche empilée.

4. Distributeur de couches selon l'une quelconque des revendications 1 à 3, **caractérisé en ce que** la paroi avant (2) a des fenêtres (11, 12).

5. Distributeur de couches selon l'une quelconque des revendications 1 à 4, **caractérisé en ce que** le récipient (1) a approximativement la forme d'un prisme droit à quatre faces.

6. Distributeur de couches selon l'une quelconque des revendications 1 à 5, **caractérisé en ce que**, pour adapter sa section interne à la pile de couches de différentes tailles, le récipient (1) a une paroi interne (6) apte à être réglée en direction de la paroi avant (2), parallèlement à la paroi arrière du récipient (1), et à être fixée.

7. Distributeur de couches selon l'une quelconque des revendications 1 à 6, **caractérisé par** un corps (11) en forme de plaque pour lester la pile de couches.

8. Distributeur de couches selon l'une quelconque des revendications 1 à 7, **caractérisé en ce qu'**au moins une des parois latérales du récipient est conformée pour fixer une console (5).
